# EUROPEAN PATENT APPLICATION

(11) **EP 2 698 168 A1**
(43) Date of publication of application: **19.02.2014**
(21) Application number: 13180235.7
(22) Date of filing: 13.08.2013
(51) Int. Cl.: A61K 47/10, A61K 9/00, A61K 31/519

(54) **Paliperidone Oral Solution**

(30) Priority: 14.08.2012 TR 201209476
(71) Applicant: Sanovel Ilac Sanayi ve Ticaret A.S., 34460 Istanbul (TR)
(72) Inventor: Cifter, Ümit, 34460 Istanbul (TR); Türkylmaz, Ali, 34460 Istanbul (TR); Mutlu, Onur, 34460 Istanbul (TR)
(74) Representative: Sevinç, Erkan

(57) **Abstract**

The present invention concerns pharmaceutical solution for use in the treatment of schizophrenia comprising paliperidone suitable for oral administration to a mammal.

## Description

### Technical Aspect

The present invention concerns pharmaceutical solution for use in the treatment of schizophrenia comprising paliperidone suitable for oral administration to a mammal.

### Background of the Present Invention

Paliperidone corresponding to the compound 4H-Pyrido[1,2-a]pyrimidin-4-one,3-[2-[4-(6-fluoro-1,2-benzisoxazol-3-yl)-1-piperidinyl]ethyl]-6,7,8,9-tetrahydro-9-hydroxy-2-methyl is an antipsychotic agent which is approved for the treatment of schizophrenia including acute treatment and recurrence prevention. Paliperidone, first described in US5254556A, is available as Invega® Extended Release Tablet and as Xeplion® as sustained release suspension for injection.

Besides, many solid oral dosage forms are disclosed in prior art. EP2079446 is about a tablet comprising a first layer of paliperidone with at least one release controlling polymer and an outer layer coating partially this first layer. Furthermore, EP2326312 discloses solid pharmaceutical composition comprising at least one solid matrix particle of paliperidone which is a monolithic matrix system.

Solid oral dosage forms have the largest and most important role in the entire pharmaceutical formulations. However, number of patients who have difficulties swallowing these dosage forms is not less. Especially, for psychiatric patients who reject to take medicine, therapeutic agents can be administrated in different forms such as solutions, suspensions, elixirs and emulsions.

US Patent No US5254556A discloses oral solution of paliperidone with pharmaceutically acceptable excipients as glycerin, parabens, tartaric acid and sorbitol. This oral solution is unbuffered and comprises only sorbitol as a stabilizing agent. Furthermore, water is the only solubilizing agent in this solution. It is already known that paliperidone is practically insoluble in water. That is the reason why the solubility of paliperidone needs to be taken into consideration when preparing an oral solution. In addition to the solubility, paliperidone needs to be physically and chemically stable in the oral solution.

### Object of the Present Invention

The main object of the present invention is to obtain an oral pharmaceutical formulation of paliperidone in solution form wherein paliperidone and excipients are dissolved homogeneously.

Another object of the present invention is to obtain an oral pharmaceutical solution providing rapid and high absorption of paliperidone which is already dissolved in the solution. It is known that absorption occurs when drugs are in a dissolved state, thus it is frequently observed that the bioavailability of oral solution is better than solid dosage forms.

Another object of the present invention is to provide a stable oral pharmaceutical solution of paliperidone.

There is also provided an oral pharmaceutical solution of paliperidone which can be administered orally to patients who have difficulty in swallowing.

There is also provided an oral pharmaceutical solution of paliperidone wherein flexible dosing is possible.

There is also provided an oral taste masked pharmaceutical solution of paliperidone.

### Description of the Present Invention

The present invention concerns pharmaceutical solution for use in the treatment of schizophrenia comprising paliperidone suitable for oral administration to a mammal.

The present invention concerns also a homogeneous, bioavailable and stable pharmaceutical solution of paliperidone suitable for oral administration to a mammal.

The term "paliperidone" as used herein refers to paliperidone as well as pharmaceutically acceptable salts, enantiomers, hydrates, solvates, metabolites, prodrugs and mixture thereof. The term also includes all polymorphic forms, whether crystalline or amorphous.

According to an embodiment of the present invention, said oral pharmaceutical solution contains paliperidone in an amount of 0.01% to 2% of total weight of the pharmaceutical solution, more preferably 0.02% to 0.5% of total weight of the pharmaceutical solution.

According to a preferred embodiment, the present invention concerns a pharmaceutical solution for oral administration to a mammal comprising: a) paliperidone as an active agent or a pharmaceutically acceptable salt thereof; and b) at least one solubilizing agent.

Solubilizing agent used according to the present invention is selected from the group comprising, but not limited to, water, dimethylisosorbide, glycols, ethyl alcohol, cetyl alcohol, glyceryl stearate, isopropyl alcohol, diethylamine, glyceryl oleate, myristyl alcohol, gelatin, cyclodextrin, polyvinyl pyrrolidone (Povidone), benzyl alcohol, glycerin, maltitol, xylitol, inositol, mannitol, invert sugars, sorbitol and the like or combination thereof. In principle, the term "glycols" as used herein refers to the group comprising ethylene glycol, polyethylene glycol, polypropylene glycol and propylene glycol. In the present invention, the preferred solubilizing agent is propylene glycol, ethyl alcohol and glycerin or combination thereof and the most preferred solubilizing agent is propylene glycol.

It has been surprisingly found that a stable and homogenous solution of paliperidone can be prepared with at least one kind of glycols as a solubilizing agent to provide a better bioavailability. Furthermore, glycols enhance physical and chemical stability of paliperidone in the present oral solution. The pharmaceutical formulation disclosed herein is a stable solution wherein paliperidone or pharmaceutically acceptable excipients are not precipitated.

According to an embodiment of the present invention, said oral pharmaceutical solution of paliperidone may also comprise an additional solubilizing agent. This additional solubilizing agent can be selected from the group of solubilizing agents and is preferably glycerin. It has also been found that a combination of glycol and glycerin in addition to its enhancing property for the solubility of paliperidone, has preservative properties wherein physical and chemical stability is also enhanced and microbial bioburden is controlled.

According to a preferred embodiment, the present invention concerns a pharmaceutical solution for oral administration to a mammal comprising: a) paliperidone as an active agent or a pharmaceutically acceptable salt thereof; and b) glycol and glycerin as a solubilizing agent. The combination of glycol and glycerin provide an increased solubility of paliperidone within the formulation. As a further embodiment of the invention, glycol is present in the range of %1 to %60 and preferably %5 to %50 by weight of total pharmaceutical solution and glycerin is present in the range of %5 to %90 and preferably %10 to %80 by weight of total pharmaceutical solution. As a further embodiment of the invention, the weight ratio of glycol to glycerin is 1:10 to 10:1 by weight of total pharmaceutical solution, preferably 1:15 to 5:1 by weight of total pharmaceutical solution. In prior art, glycerin is also known as viscosity enhancing agent and its high viscosity may slow its dissolving property. By the addition of glycerin with glycol in the ratio suitable for the present invention, pharmaceutical solution has an optimum viscosity value. And this viscosity value doesn't influence negatively solubility of the pharmaceutical solution.

According to a preferred embodiment of the present invention, said oral pharmaceutical solution of paliperidone may also include ethyl alcohol as an additional solubilizing agent. As a further embodiment of the invention, ethyl alcohol may be in the range of 2% to 20% by weight of total pharmaceutical solution.

According to a preferred embodiment of the present invention, said oral pharmaceutical solution of paliperidone may also include one or more surfactant to enhance the solubility and stability of paliperidone. Said surfactant is selected from the group comprising, but not limited to, poloxamer, polysorbate, polysorbate 20, polysorbate 40, polyoxyl 60 stearate, polysorbate 80, polysorbate 85, polysorbate 60, poloxamer 331, polyoxyethylene fatty acid esters, polyoxyl 40 castor oil, polyoxyl 40 hydrogenated castor oil, oleic acid, sodium desoxycholate, sodium lauryl sulfate, sorbitan monolaurate, sorbitan monooleate, sorbitan monopalmitate, sorbitan trioleate, myristic acid, stearic acid, sucrose stearate, tocopherol, polyoxyl castor oil, triglyceride, calcium stearate, magnesium stearate, vitamin E, propylene glycol alginate or propylene glycol laurate. The surfactant is preferably one kind of polysorbate and more preferably it is polysorbate 80. As a further embodiment of the invention, one or more surfactant may be in the range of 0.01 to 10 % and preferably 0.1 % to 5% by weight of total pharmaceutical solution.

According to a preferred embodiment of the present invention, said oral pharmaceutical solution of paliperidone have a pH from 2 to 8 and preferably from 3 to 7.5. The pH range of said solution optimizes the solubility of the therapeutic agent by increasing the ionization. Control of the pH in the solution is achieved using a buffer. As a further embodiment of the present invention, said oral pharmaceutical solution may also include one or more buffering agent for maintaining pH in the range suitable for the present invention. Buffering agent is selected from the group comprising but not limited to, alkali metal citrate, citric acid/sodium citrate, potassium hydrogen tartrate, sodium hydrogen tartrate, potassium hydrogen phthalate, sodium hydrogen phthalate, potassium dihydrogen phosphate, disodium hydrogen phosphate, hydrochloric acid/sodium hydroxide or combination thereof. Buffering agent in the present invention is preferably citric acid/sodium citrate. The amount of buffering agents used may be in the range of 0.0 1 % to 5% by weight of the total pharmaceutical solution.

According to an embodiment of the present invention, said oral pharmaceutical solution of paliperidone may also include one or more additional excipients such as sweetening agents, flavouring agents, preservatives and the like.

Sweetening agent, to mask the bitter taste of paliperidone and other excipients, is selected from the group comprising sucrose, fructose, dextrose, maltose, glucose, glycerin, lactilol, maltitol, xylitol, sorbitol, mannitol, aspartame, saccharin, neotame, acesulfame potassium, sodium cyclamate and the like or combination thereof. The amount of sweetening agents used may be in the range of 0.001% to 1% by weight of the total pharmaceutical solution.

Flavouring agent is selected from peppermint, spearmint, lime, apple, pear, peach, raspberry, plum, pineapple flavour and the like or combination thereof. The amount of flavouring agents used may be in the range of 0.01% to 1% by weight of the total pharmaceutical solution.

According to an embodiment of the present invention, said oral pharmaceutical solution of paliperidone may also include one or more preservative to prevent the growth of micro-organisms such as bacteria, yeasts and fungi in the solution. Preservative is selected from the group comprising sodium benzoate, potassium sorbate, benzyl alcohol, parabens such as methylparaben, ethylparaben, propylparaben, butylparaben and the like or combination thereof. The amount of preservatives used may be in the range of 0.01% to 1% by weight of the total pharmaceutical solution.

According to an embodiment of the present invention, said oral pharmaceutical solution comprises, paliperidone as an active agent or a pharmaceutically acceptable salt thereof; the combination of propylene glycol and glycerin as solubilizing agents, wherein the weight ratio of glycol to glycerin is 1:15 to 5:1; polysorbate 80 as surfactant; and citric acid/sodium citrate as buffering agent to maintain the pH of the solution in the range of 3 to 7.5.

According to an embodiment of the present invention, said oral pharmaceutical solution of paliperidone comprises:
a) 0.01 to 2 % by weight of paliperidone as an active agent,
b) 5 to 80 % by weight of at least one solubilizing agent,
c) 5 to 80 % by weight of at least one additional solubilizing agent,
d) 0.01 to 10 % by weight of at least one surfactant,
e) 0.01 to 5 % by weight of at least one buffering agent,
f) 0.001 to 1 % by weight of at least one sweetening agent,
g) 0.01 to 1 % by weight of at least one flavoring agent,
h) 0.01 to 1 % by weight of at least one preservative.

According to a preferred embodiment of the present invention, said oral pharmaceutical solution of paliperidone may be prepared by the process comprising the following steps:
a) Mixing and heating at least one solubilizing agent,
b) Mixing paliperidone and at least one sweetening agent to the mixture obtained,
c) Adding water, at least one preservative and one solubilizing agent,
d) Dissolving the mixture obtained,
e) Mixing at least one surfactant, one buffering agent and another sweetening agent to the mixture obtained,
f) Cooling the mixture to 30°C,
g) Mixing at least one flavoring agent,
h) Adjusting the pH to the range 2 to 8 with hydrochloric acid/sodium hydroxide buffer system.

### Examples

### Example 1

| **Agents** | **Amount (% by weight of the total solution)** |
|---|---|
| Paliperidone | 0.03% to 0.24% |
| Glycerin | 10% to 80% |
| Propylene glycol | 5% to 50% |
| Polysorbate | 0.1% to 2% |
| Citric acid | 0.2% to 1% |
| Sodium citrate | 0.02% to 0.5% |
| Maltitol | 10% to 80% |
| Sucralose | 0.001% to 0.1% |
| Methyl paraben | 0.015% to 0.3% |
| Propyl paraben | 0.01% to 0.1% |
| Flavoring | 0.01% to 0.05% |
| Water | q.s. |
| HCl/NaOH | q.s |

Glycerin and propylene glycol are mixed in container and are heated to 70ºC. To this mixture, paliperidone and maltitol are added. The solution is mixed. Water and respectively methyl paraben, propyl paraben are added to the mixture which will be dissolved by mixing. Polysorbate 80, sodium citrate, citric acid and sucralose are added to the mixture obtained. The mixture is cooled to 30ºC. Flavoring is added to the mixture cooled. The pH of the solution is adjusted with hydrochloric acid/sodium hydroxide buffer system.

### Example 2

| **Agents** | **Amount (% by weight of the total solution)** |
|---|---|
| Paliperidone | 0.03% to 0.24% |
| Glycerin | 10% to 80% |
| Propylene glycol | 5% to 50% |
| Ethyl alcohol | 2% to 20% |
| Polysorbate | 0.1% to 2% |
| Citric acid | 0.2% to 1% |
| Sodium citrate | 0.02% to 0.5% |
| Maltitol | 10% to 80% |
| Sucralose | 0.001% to 0.1% |
| Methyl paraben | 0.015% to 0.3% |
| Propyl paraben | 0.01% to 0.1% |
| Flavoring | 0.01% to 0.05% |
| Water | q.s. |
| HCl/NaOH | q.s |

Glycerin and propylene glycol are mixed in container and are heated to 70ºC. To this mixture, paliperidone and maltitol are added. The solution is mixed. Water and respectively methyl paraben, propyl paraben and ethyl alcohol are added to the mixture which will be dissolved by mixing. Polysorbate 80, sodium citrate, citric acid and sucralose are added to the mixture obtained. The mixture is cooled to 30ºC. Flavoring is added to the mixture cooled. The pH of the solution is adjusted with hydrochloric acid/sodium hydroxide buffer system.

## Claims

1. A pharmaceutical solution for oral administration to a mammal comprises:
a. paliperidone as an active agent or a pharmaceutically acceptable salt thereof; and
b. at least one glycol as solubilizing agent.

2. The pharmaceutical solution according to claim 1, wherein paliperidone is in an amount of 0.0 1 % to 2% of the total weight of the pharmaceutical solution.

3. The pharmaceutical solution according to claim 1, wherein glycol is in the range of 1% to 60% and preferably 5% to 50% by weight of the total pharmaceutical solution.

4. The pharmaceutical solution according to claim 1, wherein glycol is selected from the group comprising ethylene glycol, polyethylene glycol, polypropylene glycol and propylene glycol.

5. The pharmaceutical solution according to claim 4, wherein glycol is propylene glycol.

6. The pharmaceutical solution according to claim 1, further comprising an additional solubilizing agent.

7. The pharmaceutical solution according to claim 6, wherein the additional solubilizing agent is glycerin.

8. The pharmaceutical solution according to any preceeding claims, wherein the weight ration of glycol to glycerin is 1:10 to 10:1 and preferably 1:15 to 5:1.

9. The pharmaceutical solution according to claim 1, further comprising one or more surfactant.

10. The pharmaceutical solution according to claim 9, wherein the surfactant is in the range of 0.01% to 10% and preferably 0.1% to 5% by weight of the total pharmaceutical solution.

11. The pharmaceutical solution according to claim 9, wherein the surfactant is selected from the group comprising poloxamer, polysorbate, polysorbate 20, polysorbate 40, polyoxyl 60 stearate, polysorbate 80, polysorbate 85, polysorbate 60, poloxamer 331, polyoxyethylene fatty acid esters, polyoxyl 40 castor oil, polyoxyl 40 hydrogenated castor oil, oleic acid, sodium desoxycholate, sodium lauryl sulfate, sorbitan monolaurate, sorbitan monooleate, sorbitan monopalmitate, sorbitan trioleate, myristic acid, stearic acid, sucrose stearate, tocopherol, polyoxyl castor oil, triglyceride, calcium stearate, magnesium stearate, vitamin E, propylene glycol alginate or propylene glycol laurate.

12. The pharmaceutical solution according to claim 11, wherein the surfactant is polysorbate 80.

13. The pharmaceutical solution according to claim 1, further comprising one or more buffering agent to maintain pH of the solution in the range of 2 to 8.

14. The pharmaceutical solution according to claim 13, wherein the pH of the solution is in the range of 3 to 7.5.

15. The pharmaceutical solution according to claim 13, wherein one or more buffering agent is in the range of 0.01% to 5% by weight of the total pharmaceutical solution.

16. The pharmaceutical solution according to claim 13, wherein one or more buffering agent is selected from the group comprising alkali metal citrate, citric acid/sodium citrate, potassium hydrogen tartrate, sodium hydrogen tartrate, potassium hydrogen phthalate, sodium hydrogen phthalate, potassium dihydrogen phosphate, disodium hydrogen phosphate or combination thereof.

17. The pharmaceutical solution according to claim 16, wherein buffering agent is citric acid/sodium citrate.

18. The pharmaceutical solution according to any preceeding claims, comprising:
a. paliperidone as an active agent or a pharmaceutically acceptable salt thereof;
b. the combination of propylene glycol and glycerin as solubilizing agents, wherein the weight ratio of glycol to glycerin is 1:15 to 5:1;
c. polysorbate 80 as surfactant; and
d. citric acid/sodium citrate as buffering agent to maintain the pH of the solution in the range of 3 to 7.5.

19. The pharmaceutical solution according to any preceeding claims, further comprising one or more additional excipients selected from sweetening agents, flavouring agents and preservatives.
